## Europäisches Patentamt

(19) **European Patent Office**

Office européen des brevets

(11) Veröffentlichungsnummer **0 009 578 B1**

(12) **EUROPÄISCHE PATENTSCHRIFT**

(45) Veröffentlichungstag der Patentschrift
04.11.81

(51) Int. Cl.³ **C 12 N 11/08**

(21) Anmeldenummer 79102954.9

(22) Anmeldetag 14.08.79

(54) Verfahren zur Herstellung von an Makromoleküle gebundenen, ein Adeninringsystem enthaltenden Koenzymen und enzymatisches Verfahren.

(30) Priorität 22.09.78 DE 2841414

(43) Veröffentlichungstag der Anmeldung
16.04.80 Patentblatt 80/8

(45) Bekanntmachung des Hinweises auf die Patenterteilung:
04.11.81 Patentblatt 81/44

(84) Benannte Vertragsstaaten
CH FR GB IT NL SE

(56) Entgegenhaltungen.
**Chemical Abstracts Band 83, Nr. 17, 1975
Columbus, Ohio, USA
P. ZAPELLI et al. »Synthesis of coenzymically active soluble and insoluble macromolecularized NAD⁺ derivatives«
Seite 207, Spalte 1, Abstract Nr. 55160w
Chemical Abstracts Band 86, Nr. 13, 1977
Columbus, Ohio, USA
P. ZAPELLI et al. »Preparation and coenzymic activity of soluble polyethyleneimine-bound NADP⁺ derivatives«
Seite 182, Spalte 2 und Seite 183, Spalte 1, Abstract Nr. 85301x**

(73) Patentinhaber: **Gesellschaft für Biotechnologische Forschung mbH (GBF), Mascheroder Weg 1, D-3300 Braunschweig-Stöckheim (DE)**

(72) Erfinder: **Bückmann, Andreas, Dipl.Ing., Kutheweg 4, D-3300 Braunschweig-Stöckheim (DE)**

(74) Vertreter: **Boeters, Hans Dietrich, Dr. et al, Rumfordstrasse 40, D-8000 München 5 (DE)**

## Verfahren zur Herstellung von an Makromoleküle gebundenen, ein Adeninringsystem enthaltenden Koenzymen und enzymatisches Verfahren

AMP, ADP, ATP, NAD·/NADH und NADP·/NADPH, die kovalent an feste Träger oder an lösliche Polymere, z. B. wasserlösliche Polymere, gebunden sind, werden bereits in großem Umfang verwendet bzw. untersucht. So werden die an feste Träger gebundenen Koenzyme mit Erfolg als Liganden bei der Affinitätschromatographie verwendet. Wasserlösliche Polymere mit kovalent gebundenen Koenzymen sind bei der Affinitätsverteilung sehr brauchbar. Im Fall von NAD·/NADH und NADP·/NADPH lassen sich die entsprechenden Verbindungen regenerieren und demgemäß in enzymatischen Systemen unter Kreislaufführung verwenden.

Bekanntlich geht man bei der Herstellung von an Makromoleküle kovalent gebundenen, ein Adeninringsystem enthaltenden Koenzymen so vor, daß man zuerst eine funktionelle Gruppe am N-Atom in 6-Stellung vorsieht und danach diese reaktive Gruppe mit einem Makromolekül umsetzt.

Dabei setzt man zuerst das Adeninringsystem am N(1)-Atom mit einem Alkylierungsmittel um, das eine weitere funktionelle Gruppe trägt, die die Umsetzung mit dem Makromolekül oder eine Kettenverlängerung vor der Umsetzung mit dem Makromolekül ermöglichen soll. Als Alkylierungsmittel kommen beispielsweise Halogencarbonsäuren, wie Jodessigsäure, Epoxide, wie 3,4-Epoxybuttersäure, Lactone, wie Propiolacton, oder Aziridine, wie Äthylenimin, in Betracht.

Im Fall von AMP, ADP und ATP folgt unmittelbar auf die Alkylierung eine Dimroth-Umlagerung zur $N^6$-Form. An die Dimroth-Umlagerung kann sich die Umsetzung mit dem Makromolekül unmittelbar oder nach vorhergehender Verlängerung der Kette am N-Atom in 6-Stellung anschließen. Im Fall von NAD· und NADP· wird vor der Dimroth-Umlagerung eine Reduktion und nach der Dimroth-Umlagerung eine Reoxidation durchgeführt.

Die auf diese Weise gebildeten Koenzymderivate können für die Verknüpfung mit Makromolekülen z. B. folgende funktionelle Gruppen an Ketten am N-Atom in 6-Stellung aufweisen: Carboxyl-, Amino- oder Vinylgruppen.

Für die Verknüpfung kommen solche unlöslichen Makromoleküle (Träger, Matrizes) oder löslichen, insbesondere wasserlöslichen Makromoleküle in Betracht, die über eine oder mehrere für die Verknüpfung wirksame funktionelle Gruppen verfügen. Diese Makromoleküle können per se derartige funktionelle Gruppen aufweisen oder die funktionellen Gruppen können in die Makromoleküle eingeführt werden, z. B. nach der Bromcyan-methode. (Von der Verknüpfbarkeit der Makromoleküle kann sich der Fachmann ohne weiteres durch einen einfachen Versuch überzeugen.) Beispiele für verwendbare Makromoleküle sind: Dextrane, Polyätherpolyole, wie Polyäthylenglykol, Polyäthylenimine, Polyacrylamide, Mischpolymere, wie Poly-(Methylvinyläther/Maleinsäureanhydrid), Agarose, Glas, Zellulose und Derivate der Makromoleküle; vgl. Lowe, TIBS, 134 (1978), und Mosbach, Advances in Enzymology, 46, 205 bis 278 (1978).

Bei diesen bekannten Verfahren hat jedoch die nach der Dimroth-Umlagerung (und der gegebenenfalls folgenden Verlängerung der Kette am N-Atom in 6-Stellung) erzielbare Ausbeute bisher noch nicht befriedigt. Der folgenden Tabelle lassen sich nämlich Ausbeuten in der Größenordnung von lediglich 12 bis 40% entnehmen.

# 0 009 578

Ausbeute (%) nach Alkylierung und Dimroth-Umlagerung

| | ATP | ADP | AMP | NAD+ | NADP+ |
|---|---|---|---|---|---|
| Lindberg et al., Eur. J. Biochem., 53, 481–486 (1975) | 40*) | 40*) | 40*) | | |
| Lindberg et al., Eur. J. Biochem., 40, 187–193 (1973) | | | | 23 21*) | |
| Zappelli et al., Eur. J. Biochem., 54, 475–482 (1975) | | | | 20*) | |
| Schmidt et al., Eur. J. Biochem., 67, 295–302 (1976) | | | | 20 | |
| Lowe et al., Eur. J. Biochem., 49, 511–520 (1974) | | | | | 15–25 12–20*) |

*) Nach Verlängerung der Kette am N-Atom in 6-Stellung.

Aufgabe der Erfindung ist es, ein Verfahren zur Herstellung von an lösliche Makromoleküle oder unlösliche Makromoleküle bzw. Träger gebundenen, ein Adeninringsystem enthaltenden Koenzymen vorzusehen, bei dem man das Adeninringsystem in 1-Stellung alkyliert, das alkylierte Koenzym (gegebenenfalls mit vorhergehender Reduktion und späterer Reoxidation) einer Dimroth-Umlagerung (und gegebenenfalls einer Verlängerung der durch die Alkylierung eingeführten Alkylenkette) unterwirft und an ein Makromolekül oder einen Träger addiert und bei dem man bessere Ausbeuten erhält. Das erfindungsgemäße Verfahren ist dadurch gekennzeichnet, daß man die Dimroth-Umlagerung nach der Addition an das Makromolekül durchführt.

In 1-Stellung substituiertes AMP, ADP, ATP, NAD+ und NADP+ kann man in Ausbeuten von etwa 60 bis 90% in Abhängigkeit von der Reaktionsdauer erhalten. Diese Derivate kann man danach reinigen. Erfindungsgemäß werden die erhaltenen, in 1-Stellung substituierten Derivate an Makromoleküle bzw Träger (nachstehend kurz: Makromoleküle) addiert, wonach man die erhaltenen Makromolekülderivate von den unumgesetzten, in 1-Stellung substituierten Verbindungen abtrennen kann, die man danach erneut verwenden kann. Erst danach führt man also erfindungsgemäß die Dimroth-Umlagerung durch, der bei den NAD+- und NADP+-Derivaten eine Reduktion vorhergehen und auf die in diesen Fällen eine Reoxidation folgen kann. Da bei der Dimroth-Umlagerung praktisch kein Ausbeuteverlust eintritt, werden die Umlagerungsprodukte praktisch mit der Ausbeute der in 1-Stellung substituierten Ausgangsprodukte erhalten, d. h. mit etwa 60 bis 90%.

Die Offenbarung der vorliegenden Erfindung umfaßt auch den Inhalt der angeführten Druckschriften, insbesondere auch hinsichtlich der fakultativen Reduktion und Reoxidation im Zusammenhang mit der Dimroth-Umlagerung.

Im Rahmen der vorliegenden Erfindung wurde noch folgende Feststellung getroffen. Bei enzymatischen Verfahren in Gegenwart von Koenzymen ist die chemische Stabilität der Koenzyme ein wesentlicher Parameter. So ist NADH in wässeriger Lösung bei mäßig basischen Bedingungen (pH größer gleich 7,0 und kleiner gleich 9,0), die für eine optimale katalytische Aktivität von Dehydrogenasen erforderlich sind, recht stabil. Demgegenüber zeigt freies NAD+ bei der Verwendung im angegebenen pH-Bereich bei Raumtemperatur einen beträchtlichen Aktivitätsverlust.

Erfindungsgemäß wurde nun festgestellt, daß NAD+ in mäßig basischem Milieu gegen einen basekatalysierten Abbau stabilisiert werden kann, wenn es erfindungsgemäß kovalent an Makromoleküle gebunden ist. Es wird ein pH-Bereich von 6,0 bis 11,0, insbesondere 7,0 bis 9,0, bevorzugt, in dem Dehydrogenasen ihre optimale katalytische Aktivität zeigen.

Nachstehend wird die Erfindung durch Beispiele näher erläutert.

3

## Beispiel 1

### Herstellung von Dextran-N[6]-(Aminoäthyl)-NAD ·

### A. Herstellung von N(1)-(Aminoäthyl)-NAD ·

Es wurden 7 g NAD · (Qualität II von Boehringer) in 12 ml destilliertem Wasser gelöst. Dazu wurde 1 ml Äthylenimin (Fluka) langsam zugegeben, wobei der pH bei 4,3 bis 4,7 mit 70%iger Perchlorsäure gehalten wurde (Gesamtvolumen 19 ml). Die Reaktionsmischung wurde 3 Tage lang bei 20 C bei einem pH von 4,3 bis 4,7 (Einstellung mit 70%iger Perchlorsäure) gerührt. Die Umwandlung wurde enzymatisch mit Bierhefe-Alkoholdehydrogenase ermittelt (die enzymatische Reduktion von NAD ist im Vergleich zur Reduktion von N(1)-(Aminoäthyl)-NAD · sehr schnell). Die Reaktionsmischung wurde mit destilliertem Wasser auf 100 ml verdünnt und mit der 8- bis 10fachen Menge kaltem (0 C) Äthanol versetzt. Der erhaltene Niederschlag wurde abzentrifugiert; dadurch wurden die Nukleotide abgetrennt, während das Äthylenimin in Lösung blieb. Nach 1 1/2 h langem Zentrifugieren wurden die äthanolischen Überstände verworfen. Der Niederschlag wurde mit kaltem Äthanol gewaschen und nochmals zentrifugiert. Die Niederschläge wurden in 250 ml destilliertem Wasser gelöst, und die Lösung wurde über Nacht lyophilisiert. (Trockenes Material kann ohne Zersetzung bei +4 C in trockener Atmosphäre aufbewahrt werden.)

Die mit 7 g NAD · erhaltene lyophilisierte Reaktionsmischung wurde in 600 ml Wasser gelöst und mit einem pH von 5,75 auf einen Kationenaustauscher gegeben (70 × 25 cm, Biorad 200 bis 400 Maschen, Biorex 70), der gegen eine 0,015 m Natriumcitratlösung vom pH 5,75 ins Gleichgewicht gebracht worden war. Nach dem Beschicken wurde die NAD · -Fraktion mit einer 0,015 m Natriumcitratlösung vom pH 5,75 eluiert. Nachdem das NAD · durchgelaufen war, wurde unmittelbar mit dem Eluieren mit Wasser begonnen, dessen pH mit 1 n Salzsäure auf 3,0 eingestellt worden war (10 Säulenvolumen). N(1)-(Aminoäthyl)-NAD · und ein Nebenprodukt konnten mit 0,2 n Lithiumchlorid bzw. 0,6 m Lithiumchlorid in Wasser beim pH 3 eluiert werden. Die Fraktionen wurden unter reduziertem Druck (flash evaporation) konzentriert und viermal in kaltem Äthanol (20facher Überschuß auf Volumenbasis) gefällt, wodurch das Lithiumchlorid abgetrennt werden konnte, das in Äthanol gut löslich ist. Die Produkte ließen sich lyophilisieren und wurden in der angegebenen Weise aufbewahrt.

Die erzielten Umsätze sind im folgenden zusammengestellt.

Reaktionsergebnisse bei Umsetzung von NAD[+] mit Athylenimin (0,53 : 1 Mol)

| Reaktionsdauer (d) | Umwandlung | NAD[+] | N(1)-(Aminoäthyl)-NAD· | Nebenprodukt |
|---|---|---|---|---|
| | (enzymatisch bestimmt) | | | |
| 3 | 65 | 35 | 60 | 5 |
| 4 | 80 | 20 | 70 | 10 |

N(1)-(Aminoäthyl)-NAD[+] und das Nebenprodukt geben mit Ninhydrin eine positive Reaktion (primäre Aminogruppen sind vorhanden); beide zeigen eine charakteristische Schulter im Bereich von 300 bis 310 nm beim pH 11,5 (Alkylierung in Stellung 1 im Adeninringsystem). Mit CN[-] wird ein Komplex mit einem Absorptionsmaximum bei 325 nm gebildet (es liegt oxidiertes Nikotinamid vor). Das Nebenprodukt zeigt eine Fluoreszenz bei 366 nm (excitation). Beide sind im Test mit Bierhefe-Alkoholdehydrogenase aktiv. Die genaue Struktur des Nebenproduktes ist unbekannt.

### B. Kuppeln von N(1)-(Aminoäthyl)-NAD[+] an Makromoleküle

N(1)-(Aminoäthyl)-NAD[+] wurde an carboxyliertes Dextran gekuppelt. Die Kupplung erfolgte mit Hilfe der Carbodiimidmethode gemäß u. a. Cuatrecasas, J. Biol. Chem., 245, 3059 (1970); der Offenbarungsgehalt dieser Druckschrift wird hier miteinbezogen.

Die N(1)-(Aminoäthyl)-NAD[+]-Polymeren dieses Beispiels und der Beispiele 2 und 3 zeigten die spektralen Peaks des freien N(1)-(Aminoäthyl)-NAD[+], und zwar ein Absorptionsmaximum bei 259 nm, eine Schulter im Bereich von 300 bis 310 nm beim pH 11,5 und mit CN[-] die Absorption des Komplexes bei 325 nm.

### C. Herstellung von Dextran-N(1)-(Aminoäthyl)-NADH

Es wurde von 15 ml gelfiltriertem Dextran-N(1)-Aminoäthyl)-NAD$^+$ ausgegangen, wobei 10 Moleküle N(1)-(Aminoäthyl)-NAD$^+$ auf 1 Molekül Dextran kamen und das mittlere Molekulargewicht des Dextrans 40 000 Dalton betrug. Die Konzentration war 4,9 mmol mit insgesamt 73,5 $\mu$Mol N(1)-(Aminoäthyl)-NAD$^+$ auf Basis von epsilon$_{259}$ = 18 500 M$^{-1}$cm$^{-1}$. Die Verbindung wurde mit einem zehnfachen Überschuß von $Na_2S_2O_4$ in 5 min bei 70°C in Gegenwart von Natriumhydrogencarbonat (1%) im pH-Bereich von 7,5 bis 8,0 reduziert. Es wurden 16 ml mit einer 4,5 mmol. Konzentration und einer Gesamtmenge von 72 $\mu$Mol (berechnet als N(1)-(Aminoäthyl)-NADH) auf Basis von epsilon$_{340}$ = 6220 M$^{-1}$cm$^{-1}$ erhalten.

Das Hauptabsorptionsmaximum des gebildeten Dextran-N(1)-(Aminoäthyl)-NADH lag bei 259 nm und ein zweites Maximum bei 340 nm, das die Anwesenheit von reduziertem Nikotinamid anzeigte. Verstärkte Absorption im Bereich von 300 bis 310 nm beim pH 11,5, was eine Alkylierung in 1-Stellung des Adeninringsystems anzeigte. Absorption bei 259 nm/Absorption bei 340 nm = 2,4. $\rightarrow$ epsilon$_{259}$ = ± 15 000 M$^{-1}$cm$^{-1}$.

### D. Herstellung von Dextran-N$^6$-(Aminoäthyl)-NADH

Durch die Reaktionsmischung der Stufe C leitete man 10 min lang Sauerstoff, um überschüssiges $Na_2S_2O_4$ zu oxidieren. Danach wurde die Dimroth-Umlagerung durchgeführt, wobei man 1,25 h lang auf 70°C beim pH 11,0 erhitzte.

Danach wurde eine Gelfiltration auf einer Säule (70 × 3,5 cm, Sephadex G 50) in 0,03 m Kaliumchlorid beim pH 10 durchgeführt. Das Konzentrieren der Fraktion des Dextran-Nukleotid-Materials lieferte 23 ml eines 3,14 mmol. Konzentrats mit insgesamt 72,2 $\mu$Mol (berechnet als N$^6$-(Aminoäthyl)-NADH) gemäß epsilon$_{340}$ = 6220 M$^{-1}$cm$^{-1}$.

Das Hauptabsorptionsmaximum lag bei 267 nm und ein zweites Maximum bei 340 nm. Keine Erhöhung der Absorption im Bereich von 300 bis 310 nm beim pH 11,5, was anzeigte, daß die 1-Stellung nicht mehr alkyliert war. Absorption bei 267 nm/Absorption bei 340 nm = 3,7. $\rightarrow$ epsilon$_{267}$ = 23 000 M$^{-1}$cm$^{-1}$.

Das Derivat zeigte koenzymatische Aktivität bei Tests mit Dehydrogenasen, wie Alanin-, Alkohol-, Lactat-, Glutamat- und Maleatdehydrogenasen, und konnte enzymatisch bis zu mehr als 95% oxidiert werden.

### E. Oxidation zu Dextran-N$^6$-(Aminoäthyl)-NAD$^+$

Es wurden 9 ml Dextran-N$^6$-(Aminoäthyl)-NADH mit einer 6,5 mmol. Konzentration und einer Gesamtmenge von 58,5 $\mu$Mol (berechnet als reduziertes Nukleotid) auf Basis von epsilon$_{340}$ = 6220 M$^{-1}$cm$^{-1}$ mit 3 ml einer wässerigen 2 mmol. Riboflavin-Lösung und 3 ml eines 0,5 mmol. Kaliumphosphatpuffers beim pH 7,0, bei Raumtemperatur und bei Tageslicht 2 h lang umgesetzt, wobei man Sauerstoff durchleitete.

Danach wurde eine Gelfiltration auf einer Säule (70 × 3,5 cm, Sephadex G 50) mit 0,03 m Kaliumchlorid durchgeführt, wonach unter reduziertem Druck (flash evaporation) konzentriert wurde. Es wurden 17,5 ml Dextran-N$^6$-(Aminoäthyl)-NAD$^+$ mit einer 3,37 mmol. Konzentration und insgesamt 59 $\mu$Mol (berechnet als N$^6$-(Aminoäthyl)-NAD$^+$) auf Basis von epsilon$_{267}$ = 23 000 M$^{-1}$cm$^{-1}$ erhalten.

Das Hauptabsorptionsmaximum lag bei 267 nm, wobei im Bereich von 300 bis 310 nm keine Schulter auftrat. Mit CN$^-$ erhielt man einen Komplex mit einer maximalen Absorption bei 325 nm.

Das erhaltene Derivat zeigte eine koenzymatische Aktivität bei Tests mit Alkohol-, Lactat- und Formiatdehydrogenase. Es konnte eine enzymatische Reduktion bis zu 50 bis 60% erreicht werden; diese relativ geringe prozentuale Ausbeute läßt sich auf die Gleichgewichtseinstellung der katalysierten Reaktion zurückführen.

### Beispiel 2

### Herstellung von Polyäthylenglykol-N$^6$-(Aminoäthyl)-NAD$^+$

A bis B. Es wurde gemäß den Stufen A bis B des Beispiels 1 gearbeitet, wobei jedoch carboxyliertes Polyäthylenglykol verwendet wurde.

C bis E. Es wurde von 12 ml Polyäthylenglykol-N(1)-(Aminoäthyl)-NAD$^+$ mit insgesamt 64 $\mu$Mol N(1)-(Aminoäthyl)-NAD$^+$ mit einer 5,3 mmol. Konzentration auf Basis von epsilon$_{255}$ = 18 500 M$^{-1}$cm$^{-1}$ ausgegangen. Die Reduktion lieferte 13 ml Polyäthylenglykol-N(1)-(Aminoäthyl)-NADH mit insgesamt 60 $\mu$Mol N(1)-(Aminoäthyl)-NADH mit einer 4,61 mmol. Konzentration auf Basis von epsilon$_{340}$ = 6220 M$^{-1}$cm$^{-1}$ (Absorption bei 259 nm/Absorption bei 340 nm = 2,40). Nach $O_2$-Durchleiten und

Dimroth-Umlagerung erhielt man 20 ml Polyäthylenglykol-N6-(Aminoäthyl)-NADH mit insgesamt 59,8 µMol N6-(Aminoäthyl)-NADH mit einer 3 mmol. Konzentration auf Basis von epsilon$_{340}$ = 6220 M$^{-1}$cm$^{-1}$ (Absorption bei 267 nm/Absorption bei 340 nm = 3,7). Die Oxidation lieferte 26 ml Polyäthylenglykol-N6-(Aminoäthyl)-NAD$^+$ mit insgesamt 54 µMol N6-(Aminoäthyl)-NAD$^+$ und einer 2,0 mmol. Konzentration auf Basis einer 90%igen enzymatischen Rückreduktion.

### Beispiel 3

Herstellung von Poly-(Methylvinyläther/Maleinsäureanhydrid)-N6-(Aminoäthyl)-NAD$^+$

A bis B. Es wurde gemäß den Stufen A bis B des Beispiels 1 gearbeitet, wobei jedoch carboxyliertes Poly-(Methylvinyläther/Maleinsäureanhydrid) verwendet wurde.

Poly-(Methylvinyläther/Maleinsäureanhydrid bzw. (Methylvinyläther/Maleinsäureanhydrid)-Polymeres enthält Anhydridgruppen, die gegenüber Verbindungen mit primären Aminogruppen sehr reaktiv sind. Deswegen kann N(1)-(Aminoäthyl)-NAD$^+$ quantitativ (100%) beim pH 6,5 bis 7,0 bei einem bestimmten Monomer/N(1)-(Aminoäthyl)-NAD$^+$-Verhältnis (z. B. 10 bis 50 : 1 bzw. 10 : 50) gebunden werden.

C bis E. Es wurde von 11,8 ml Poly-(Methylvinyläther/Maleinsäureanhydrid)-N(1)-(Aminoäthyl)-NAD$^+$ mit insgesamt 49 µMol N(1)-(Aminoäthyl)-NAD$^+$ mit einer 4,15 mmol. Konzentration auf Basis von epsilon$_{259}$ = 18 500 M$^{-1}$cm$^{-1}$ ausgegangen. Die Reduktion lieferte 12 ml Poly-(Methylvinyläther/Maleinsäureanhydrid)-N(1)-(Aminoäthyl)-NADH mit insgesamt 47 µMol N(1)-(Aminoäthyl)-NADH mit einer 3,92 mmol. Konzentration auf Basis von epsilon$_{340}$ = 6220 M$^{-1}$cm$^{-1}$ (Absorption bei 259 nm/Absorption bei 340 nm = 2,45). Nach $O_2$-Durchleiten und Dimroth-Umlagerung erhielt man 12,5 ml Poly-(Methylvinyläther/Maleinsäureanhydrid)-N6-(Aminoäthyl)-NADH mit insgesamt 43 µMol N6-(Aminoäthyl)-NADH und einer 3,45 mmol. Konzentration auf Basis von epsilon$_{340}$ = 6220 M$^{-1}$cm$^{-1}$ (Absorption bei 267 nm/Absorption bei 390 nm = 3,9 bis 4,0). Die Oxidation lieferte 16 ml Poly-(Methylvinyläther/Maleinsäureanhydrid)-N6-(Aminoäthyl)-NAD$^+$ mit insgesamt 30 µMol N6-(Aminoäthyl)-NAD$^+$ mit einer 1,9 mmol. Konzentration auf Basis einer 70%igen enzymatischen Rückreduktion.

### Beispiel 4

Es wurden Stabilitätsuntersuchungen durchgeführt, bei denen man NAD$^+$, Poly-(Methylvinyläther/Maleinsäureanhydrid)-N6-(Aminoäthyl)-NAD$^+$, Dextran-N6-(Aminoäthyl)-NAD$^+$ (Dextran $\overline{M}$40 000 Dalton) und Polyäthylenglykol-N6-(Aminoäthyl)-NAD$^+$ (Polyäthylenglykol $\overline{M}$ 6000) aus den Beispielen 1 bis 3 bei einer 1 mmol. Nukleotidkonzentration beim pH 9 in 0,1 m Tris/HCl mit Natriumazid (0,02%) (zum Inhibieren einer möglichen mikrobiellen Kontamination) bei Raumtemperatur inkubierte. Man testete die Stabilität, indem man die enzymatische Reduzierbarkeit mit Laktat- bzw. Alkoholdehydrogenase verfolgte. Dabei setzte man die maximal erreichbare Absorption bei 340 nm zum Zeitpunkt 0 mit 100% an. Bei dem an Polymere gebundenen N6-(Aminoäthyl)-NAD$^+$ wurde außerdem eine Gelfiltration auf einer Analysensäule (Sephadex G 50) vorgenommen, um freies Nukleotid zu ermitteln. Die Bedingungen waren wie folgt:

Alkoholdehydrogenase
(Polyäthylenglykol-N6-(Aminoäthyl)-NAD$^+$):
0,1 m Tris/HCl (pH 8,0) + EDTA (10$^{-4}$ Mol)
+ Semicarbazid (7 x 10$^{-3}$ Mol), Äthanol (0,2 Mol),
Zugabe von Bierhefe-Alkoholdehydrogenase;
Laktatdehydrogenase
(NAD$^+$, Poly-(Methylvinyläther/Maleinsäureanhydrid)-N6-(Aminoäthyl)-NAD$^+$ und
Dextran-N6-(Aminoäthyl)-NAD$^+$):
0,1 m Tris/HCl (pH 9,0) + EDTA (10$^{-4}$ Mol),
Laktat (0,1 Mol), Zugabe von Laktatdehydrogenase.

Die überraschend erhöhte NAD$^+$-Stabilität bei kovalenter Bindung an Polymere läßt sich der graphischen Darstellung (Figur) als Funktion der Zeit entnehmen. Es konnte kein freis Material mit einer Absorption bei 267 nm bei der Gelfiltration selbst nach langer Inkubation bei dem an Polymere gebundenen N6-(Aminoäthyl)-NAD$^+$ festgestellt werden.

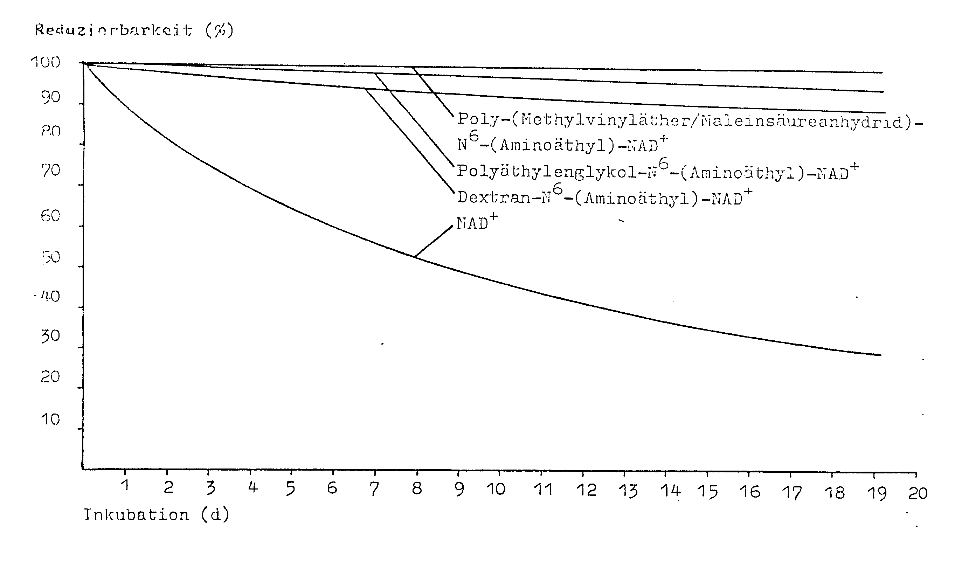

## Patentansprüche

1. Verfahren zur Herstellung von an lösliche Makromoleküle oder unlösliche Makromoleküle oder Träger kovalent gebundenen, ein Adeninringsystem enthaltenden Koenzymen, bei dem man das Adeninringsystem in 1-Stellung mit einem Alkylierungsmittel, das eine weitere funktionelle Gruppe trägt, alkyliert, das alkylierte Koenzym (gegebenenfalls mit vorhergehender Reduktion und späterer Reoxidation) einer Dimroth-Umlagerung (und gegebenenfalls einer Verlängerung der durch die Alkylierung eingeführten Kette) unterwirft und an ein Makromolekül oder einen Träger kuppelt, dadurch gekennzeichnet, daß man die Dimroth-Umlagerung nach der Kupplung an das Makromolekül durchführt.

2. Enzymatisches Verfahren unter Verwendung einer Dehydrogenase und des gemäß Anspruch 1 an Makromoleküle oder Träger kovalent gebundenen NAD⁻, dadurch gekennzeichnet, daß man bei einem pH-Wert im Bereich von 6,0 bis 11,0 arbeitet.

3. Verfahren nach Anspruch 2, dadurch gekennzeichnet, daß man bei einem pH-Wert im Bereich von 7,0 bis 9,0 arbeitet.

## Claims

1. Process for the manufacture of coenzymes that are covalently bonded to soluble macromolecules or insoluble macromolecules or carriers and contain an adenine ring system, in which the adenine ring system is alkylated in the 1-position by an alkylating agent carrying an additional functional group, the alkylated coenzyme (optionally previously reduced and later reoxidised) is subjected to a Dimroth rearrangement (and optionally to an elongation of the chain introduced by the alkylation) and added to a macromolecule or a carrier, characterised in that the Dimroth rearrangement is carried out after the addition to the macromolecule.

2. Enzymatic process using a dehydrogenase and NAD⁺ covalently bonded, according to claim 1, to macromolecules or carriers, characterised in that the process is carried out at a pH in the range of from 6.0 to 11.0.

3. Process according to claim 2, characterised in that the process is carried out at a pH in the range of from 7.0 to 9.0.

## Revendications

1. Procédé de production de co-enzymes contenant un système de cycle d'adénine, fixées par covalence sur des macromolécules solubles ou sur des macromolécules ou supports insolubles, selon lequel on alkyle le système du cycle d'adénine en position 1 avec un agent d'alkylation porteur d'un groupe fonctionnel supplementaire, on soumet la co-enzyme alkylée (éventuellement après réduction préliminaire et avec réoxydation ultérieure) à une transposition de Dimroth (et éventuellement à un allongement de la chaîne introduite par alkylation) et l'on fixe sur une macromolécule ou sur un support, procédé caractérisé en ce qu'on effectue la transposition de Dimroth après la fixation sur la macromolécule.

2. Procédé enzymatique utilisant une déshydrogénase et le NAD⁺ (nicotinamide-adénine-dinucléotide) fixé par covalence sur une macromolécule ou sur un support selon la revendication 1, caractérisé en ce qu'on travaille à un pH compris entre 6,0 et 11,0.

3. Procédé selon la revendication 2, caractérisé en ce qu'on travaille à un pH compris entre 7,0 et 9,0.

Reduzierbarkeit (%)

100
90
80
70
60
50
40
30
20
10

Poly-(Methylvinyläther/Maleinsäureanhydrid)-
$N^6$-(Aminoäthyl)-NAD$^+$

Polyäthylenglykol-$N^6$-(Aminoäthyl)-NAD$^+$

Dextran-$N^6$-(Aminoäthyl)-NAD$^+$

NAD$^+$

1  2  3  4  5  6  7  8  9  10  11  12  13  14  15  16  17  18  19  20

Inkubation (d)

0 009 578